(19) Europäisches Patentamt
European Patent Office
Office européen des brevets

(11) EP 4 417 220 A1

(12) EUROPEAN PATENT APPLICATION
published in accordance with Art. 153(4) EPC

(43) Date of publication:
**21.08.2024 Bulletin 2024/34**

(21) Application number: **22880991.9**

(22) Date of filing: **11.10.2022**

(51) International Patent Classification (IPC):
***A61K 47/26*** *(2006.01)* ***A61K 9/14*** *(2006.01)*
***A61K 9/32*** *(2006.01)* ***A61K 9/36*** *(2006.01)*
***A61K 31/4422*** *(2006.01)* ***A61K 47/02*** *(2006.01)*
***A61K 47/40*** *(2006.01)* ***A61K 47/58*** *(2017.01)*

(52) Cooperative Patent Classification (CPC):
**A61K 9/14; A61K 9/28; A61K 31/4422; A61K 47/02; A61K 47/26; A61K 47/40; A61K 47/58**

(86) International application number:
**PCT/JP2022/037812**

(87) International publication number:
**WO 2023/063296 (20.04.2023 Gazette 2023/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.10.2021 JP 2021166788**

(71) Applicant: **TOWA PHARMACEUTICAL CO., LTD.**
**Kadoma-shi,**
**Osaka 571-8580 (JP)**

(72) Inventors:
- **YUMINOKI, Kayo**
  **Kadoma-shi, Osaka 571-8580 (JP)**
- **YAMASHITA, Yoshihito**
  **Kadoma-shi, Osaka 571-8580 (JP)**
- **HONJO, Tatsuya**
  **Kadoma-shi, Osaka 571-8580 (JP)**
- **SAEKI, Isamu**
  **Kadoma-shi, Osaka 571-8580 (JP)**
- **OKUDA, Yutaka**
  **Kadoma-shi, Osaka 571-8580 (JP)**

(74) Representative: **V.O.**
**P.O. Box 87930**
**2508 DH Den Haag (NL)**

# (54) METHOD FOR PRODUCING LIQUID MIXTURE, LIQUID MIXTURE, AND SOLID COMPOSITION

(57) A method for producing a liquid mixture, the method including: a first mixing step of mixing fine particles and a hydroxy compound to obtain a hydroxy compound coated with fine particles; and a second mixing step of mixing the hydroxy compound coated with fine particles with a solvent to obtain a liquid mixture in which fine particles coated with a hydroxy compound are dispersed in the solvent.

FIG. 1

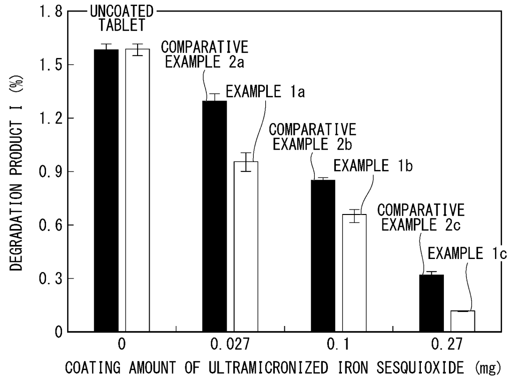


EP 4 417 220 A1

## Description

[Technical Field]

The present invention relates to a method for producing a liquid mixture, a liquid mixture, and a solid composition.
Priority is claimed on Japanese Patent Application No. 2021-166788, filed October 11, 2021, the content of which is incorporated herein by reference.

[Background Art]

In the pharmaceutical field, the food field, the cosmetic field, the coating field, and the like, from the viewpoint of improving scratch resistance, surface hardness, durability, heat resistance, light resistance, atmospheric corrosion resistance, ultraviolet shielding properties, electrical conductivity, and the like, attempts have been made to coat the surface of host particles with fine particles (guest particles) to impart new physical properties.
Specifically, in the pharmaceutical field, attempts have been made to coat tablets with metal oxides such as microparticulate titanium oxide and microparticulate iron oxide and to improve the light stability of active ingredients included in the tablets. In addition, in the cosmetic field, attempts have been made to coat microparticulate titanium oxide with a touch feeling modifier such as a nylon resin or a silicone resin and to improve ultraviolet shielding properties while not impairing touch feeling.
In recent years, from the viewpoint of further improving functions, it has been required to uniformly coat the surface of host particles with fine particles. However, in fine particles having a size of approximately several dozen μm or less, since an intermolecular force such as attractive force (for example, van der Waals force) acting between the particles, is greater than the gravity applied to the particles, the fine particles become adherent particles having high aggregability, and it is difficult to uniformly coat the surface of the host particles with the fine particles.
Examples of the method of coating the surface of host particles with fine particles include a method of dispersing the fine particles in a solution to create a coating liquid, and coating the host particles with the coating liquid. In order to uniformly coat the surface of host particles with fine particles, for example, it has been attempted to add an additive such as a dispersant to the coating liquid and suppress the aggregation of fine particles.
For example, Patent Document 1 discloses a coating agent containing fine metal particles, a dispersion medium, a fine metal particle dispersant, and a binder, in which the fine metal particles are fine particles of a metal oxide, the fine metal particle dispersant contains a monomer or oligomer having two or more active energy-curing groups and at least one carboxyl group, and the binder contains a (meth)acrylic resin binder having a (meth)acryloyl group in a side chain. It is disclosed that according to the coating agent, the dispersibility and dispersion stability of the fine metal particles are excellent, and improvement of the transparency, adhesiveness, hardness, and durability (alkali resistance and light resistance) of the coating film can be promoted.

[Citation List]

[Patent Document]

[Patent Document 1]
Japanese Patent No. 6186032

[Summary of Invention]

[Technical Problem]

However, in a conventional liquid mixture (typically, a coating liquid), the improvement of the dispersibility and dispersion stability of fine particles is not sufficient, and even when the host particles are coated by using the liquid mixture, there is room for improvement in the uniformity of the fine particles on the surface of the host particles.
In addition, even in a case where fine particles having a smaller particle diameter are used as a raw material, the fine particles aggregate during the production process for the liquid mixture, and there is also a problem in that the particle diameter of the fine particles in the liquid mixture rather becomes larger compared to the case where conventional fine particles with a larger particle diameter are used as a raw material.
The present invention has been made in view of such circumstances, and it is an object of the invention to provide a method for producing a liquid mixture in which fine particles are present with satisfactory dispersibility and dispersion stability, a liquid mixture obtained by the production method, a solid composition coated with the liquid mixture, and a solid composition created using the liquid mixture.

[Solution to Problem]

In order to solve the above-described problems, the present inventors have made extensive studies on fine particles and dispersants. As a result, the inventors found that instead of simply mixing each raw material to produce a liquid mixture, by mixing fine particles and a specific dispersant (hydroxy compound) in advance to create composite particles, and dissolving a dispersant of the composite particles in a solvent to produce a liquid mixture, a liquid mixture in which the fine particles are present with dramatically improved dispersibility and dispersion stability can be produced, thus completing the present invention.

That is, the present invention has the following aspects.

[1] A method for producing a liquid mixture, the method comprising: a first mixing step of mixing fine particles and a hydroxy compound to obtain a hydroxy compound coated with fine particles; and a second mixing step of mixing the hydroxy compound coated with fine particles with a solvent to obtain a liquid mixture in which fine particles coated with a hydroxy compound are dispersed in the solvent.
[2] The method for producing a liquid mixture according to [1], in which the hydroxy compound is one or more compounds selected from the group consisting of a sugar, a vitamin P derivative, and a salt of a vitamin P derivative.
[3] The method for producing a liquid mixture according to [1] or [2], in which a high-molecular-weight dispersant is further mixed in the second mixing step.
[4] The method for producing a liquid mixture according to any one of [1] to [3], in which the average particle diameter D50 of the fine particles in the first mixing step is 1 nm or greater and 900 nm or less, and the average particle diameter D50 of the hydroxy compound is 30 μm or greater and 300 μm or less.
[5] The method for producing a liquid mixture according to any one of [1] to [4], in which the average particle diameter D50 of the fine particles in the second mixing step is 410 nm or less.
[6] The method for producing a liquid mixture according to any one of [1] to [5], in which in the first mixing step, the use amount of the hydroxy compound is 200 parts by mass or greater and 2,500 parts by mass or less with respect to 100 parts by mass of the fine particles.
[7] A liquid mixture in which fine particles coated with a hydroxy compound are dispersed in a solvent, in which the hydroxy compound is D-mannitol, α-cyclodextrin, HP-β-cyclodextrin, methyl hesperidin, and sucralose.
[8] A solid composition coated with the liquid mixture according to [7].
[9] A solid pharmaceutical composition coated with the liquid mixture according to [7].
[10] A solid composition created by using the liquid mixture according to [7].
[11] A liquid mixture containing: a hydroxy compound; and fine particles, in which the average particle diameter D50 of the fine particles is 200 nm or less, and the span value of the fine particles is 9 or less.

[Advantageous Effects of Invention]

According to the present invention, a method for producing a liquid mixture in which fine particles are present with satisfactory dispersibility and dispersion stability, a liquid mixture obtained by the production method, a solid composition coated with the liquid mixture, and a solid composition created using the liquid mixture, can be provided.

[Brief Description of Drawings]

FIG. 1 is a graph showing results for the evaluation of light stability of solid compositions of Examples and Comparative Examples.
FIG. 2 is a graph showing results for the evaluation of light stability of solid compositions of Examples and Comparative Examples.

[Description of Embodiments]

(Method for producing liquid mixture)

The method for producing a liquid mixture of the present embodiment includes: a first mixing step of mixing fine particles and a hydroxy compound to obtain a hydroxy compound coated with fine particles (hereinafter, referred to as a "first mixing step"); and a second mixing step of mixing the hydroxy compound coated with fine particles with a solvent to obtain a liquid mixture in which fine particles coated with a hydroxy compound are dispersed in a solvent (hereinafter, referred to as a "second mixing step").

<First mixing step>

The first mixing step in the method for producing a liquid mixture of the present embodiment is a step of mixing fine particles and a hydroxy compound to obtain a hydroxy compound coated with fine particles.

Here, the "hydroxy compound coated with fine particles" is a material in which a portion or the entirety of the surface of a solid hydroxy compound is covered with fine particles.

<<Fine particles>>

In the present specification, the term "fine particles" means particles in which the average particle diameter D50 measured by a laser diffraction method is less than 1 μm.

In the present specification, the average particle diameter D50 can be measured by the following method.

First, 100 mg of fine particles are dispersed in ethanol. Next, for the fine particles dispersed in ethanol, the particle size distribution of the fine particles is obtained by using a laser diffraction/scattering-type particle diameter distribution analyzer (for example, "LA-950" manufactured by HORIBA, Ltd.). The average particle diameter D50 is calculated by determining the particle diameter at a cumulative percentage of 50% from the smaller diameter side on the basis of a cumulative volume distribution diagram of the obtained particle size distribution of the fine particles.

The average particle diameter D50 of the fine particles in the liquid mixture can be measured by the following method. Since components other than the fine particles in the liquid mixture are present in a state of being dissolved in a solvent, the liquid mixture is diluted to an appropriate concentration, and the particle size distribution of the fine particles is obtained by using a laser diffraction-type particle diameter distribution analyzer (for example, "LA-950" manufactured by HORIBA, Ltd.). The average particle diameter D50 is calculated by determining the particle diameter at a cumulative percentage of 50% from the smaller diameter side on the basis of a cumulative volume distribution diagram of the obtained particle size distribution of the fine particles.

In a case where the liquid mixture includes two or more kinds of fine particles, the average particle diameter D50 measured by the above-described method may be within the preferable range that will be described below for all the fine particles; however, it is acceptable when the average particle diameter D50 of at least one kind of fine particles is within the preferable range that will be described below.

Regarding a method of extracting only one kind from two or more kinds of fine particles, for example, in a case where the particle diameters of the fine particles are significantly different, only one kind can be extracted by sieving. In addition, one kind of fine particles can also be separated by utilizing the sedimentation velocity or the difference in specific gravity.

The fine particle used in the first step has an average particle diameter D50 of preferably 1 nm or greater and 900 nm or less, more preferably 1 nm or greater and 600 nm or less, even more preferably 10 nm or greater and 150 nm or less, and particularly preferably 10 nm or greater and 50 nm or less, from the viewpoint of further obtaining the effects of the present invention.

Regarding the fine particles used in the first step, all of organic fine particles and inorganic fine particles can be used.

In addition, the fine particles may be surface-treated or may be composite particles such as microcapsule-type fine particles.

· Organic fine particles

Examples of the organic fine particles include fine polymer particles obtained by emulsion polymerization, microemulsion-based polymerization, soap-free polymerization, seed polymerization, dispersion polymerization, suspension polymerization, or the like (for example, fine polymer particles of polyethylene, polypropylene, polystyrene, polyacrylate, polyamide, a silicone resin, a phenol resin, and a natural polymer, in the form of powder, latex, or emulsion); organic pigments (for example, an azo lake, an insoluble azo pigment, a condensed azo pigment, a chelate azo pigment, a phthalocyanine pigment, a perylene pigment, a perinone pigment, a quinacridone pigment, a thioindigo pigment, an isoindolinone pigment, a quinophthalone pigment, a dioxazine pigment, an anthraquinone pigment, a nitro pigment, a nitroso pigment, and aniline black); foods; and drugs (amlodipine, evastin, selegiline, brotizolam, ramosetron, midodrin, montelukast, azulene sulfonic acid, etizolam, bromperidol, mecobalamin, alpha-calcidol, bromocriptine, pramipexole, rosuvastatin, silodosin, nifedipine, and pharmacologically acceptable salts or solvates thereof).

More specific examples of the organic pigments include Red No. 202, Red No. 228, Red No. 226, Yellow No. 4, Blue No. 404, Yellow No. 5, Red No. 505, Red No. 230, Red No. 223, Orange No. 201, Red No. 213, Yellow No. 204, Yellow No. 203, Blue No. 1, Green No. 201, Violet No. 201, and Red No. 204, all of which may be in a lake form.

· Inorganic fine particles

Examples of the inorganic fine particles include metal oxides such as iron oxide (red iron oxide, yellow iron oxide, and black iron oxide), silicon dioxide (silica), titanium dioxide, zinc oxide, aluminum oxide, cobalt oxide, and thallium oxide; silicate minerals such as mica, talc, sericite, kaolin, and synthetic mica; metal carbonates such as calcium carbonate and magnesium carbonate; metal sulfates such as barium sulfate; metal particles of copper, silver, gold, nickel, palladium, platinum, and cobalt; composite powders such as titanated mica; boron nitride; lead white; carbon black; cadmium red; yellow lead; ultramarine blue; cobalt blue; cobalt violet; and zinc chromate.
Among those described above, from the viewpoint of having higher aggregability and more easily obtaining the effects of the present invention, the fine particles according to the present embodiment are preferably inorganic fine particles, more preferably a metal oxide, and even more preferably iron oxide or titanium dioxide.
Regarding the fine particles according to the present embodiment, one kind thereof may be used alone, or two or more kinds thereof may be used in combination.

<Hydroxy compound>

In the present specification, the term "hydroxy compound" is a compound which is different from the above-mentioned "fine particles" and means a compound having one or more hydroxy groups. In addition, in the present specification, the "hydroxy compound" means a compound which is solid at 25°C.
Specifically, the hydroxy compound used in the first step is not particularly limited as long as it is a compound that is solid at 25°C and dissolves in a solvent in the second mixing step that will be described below.
More specific examples of the hydroxy compound used in the first step include a polyhydric alcohol and a vitamin derivative.

· Polyhydric alcohol

Examples of the polyhydric alcohol include trihydric alcohols such as trimethylolethane and trimethylolpropane; tetrahydric alcohols such as pentaerythritol and erythritol; pentahydric alcohols such as arabitol and xylitol; hexahydric alcohols such as dipentaerythritol, sorbitol, mannitol, iditol, inositol, talose, and allose; and derivatives of the polyols in which some or all of the hydrogen atoms contained in these compounds have been substituted with other substituents.
Taking inositol as an example, there are nine stereoisomers of inositol, namely, cis-inositol, epi-inositol, allo-inositol, myo-inositol, muco-inositol, neo-inositol, chiro-inositol (D-form and L-form exist), and scyllo-inositol, and similarly, for other compounds as well, only one kind of isomer may be used, or two or more kinds of isomers may be used in combination.
It is more preferable that the hydroxy compound used in the first step is a sugar among the polyhydric alcohols.

· Sugar

The sugar may be a monosaccharide or an oligosaccharide. Here, the monosaccharide means a sugar that cannot be further hydrolyzed, and means a compound that serves as a constituent element when forming a polysaccharide. The monosaccharide can also be said to be the smallest unit of a sugar. The oligosaccharide is an oligomer of sugar in which a plurality of monosaccharides are bonded by glycoside bonds.

·· Monosaccharide

Specific examples of the monosaccharide include glucose (grape sugar), fructose (fruit sugar), galactose, sucralose, ribose, xylose, mannitol, sorbitol, xylitol, erythritol, and pentaerythritol.

·· Oligosaccharide

Specific examples of the oligosaccharide include disaccharides such as sucrose (cane sugar), lactose (milk sugar), maltose (malt sugar), isomaltose, trehalose, cellobiose, and maltitol; trisaccharides such as raffinose, melezitose, and maltotriose; tetrasaccharides such as stachyose; hexasaccharides such as α-cyclodextrin; heptasaccharides such as β-cyclodextrin; and octasaccharides such as γ-cyclodextrin.
The oligosaccharide may be an oligosaccharide derivative. Specific examples of the oligosaccharide derivatives include 2-hydroxypropyl-β-cyclodextrin (HP-β-cyclodextrin) obtained by 2-hydroxypropylating a hydroxy group of β-cyclodextrin.

·· Other sugars

In addition, examples of the sugar may include other sugars such as a heptose, a deoxy sugar, an amino sugar, a thiosugar, a selenosugar, an aldonic acid, a uronic acid, a sugar acid, ascorbic acid, a ketoaldonic acid, an anhydro sugar, an unsaturated sugar, a sugar ester, a sugar ether, and a glycoside, and the sugar may also be a hydrolyzate of a polysaccharide such as starch, glycogen, or cellulose.

· Vitamin derivative

Examples of the vitamin derivative include a vitamin C derivative or a salt thereof, a vitamin E derivative or a salt thereof, and a vitamin P derivative or a salt thereof.

·· Vitamin C derivative or salt thereof

Examples of the vitamin C derivatives include an ascorbic acid derivative in which at least one hydroxyl group of ascorbic acid is derivatized.
More specific examples of the ascorbic acid derivative include ascorbyl phosphate obtained by subjecting any of the hydroxyl groups of ascorbic acid to phosphoric acid esterification; ethyl ascorbic acid obtained by ethoxylating any of the hydroxyl groups of ascorbic acid; ascorbic acid glucoside obtained by glucosidizing any of the hydroxyl groups of ascorbic acid; acylated ascorbic acid obtained by acylating any of the hydroxyl groups of ascorbic acid; and glyceryl ascorbic acid obtained by substituting any of the hydroxyl groups of ascorbic acid with glycerin.
Examples of the salt of the ascorbic acid derivative include a salt of an ascorbic acid derivative with an inorganic base and a salt of an ascorbic acid derivative with an organic base.
Examples of the salt with an inorganic base include alkali metal salts such as sodium salts and potassium salts; alkaline earth metal salts such as calcium salts and magnesium salts; aluminum salts; ammonium salts; and zinc salts.
Examples of the salt with an organic base include alkylammonium salts and salts with basic amino acids.

·· Vitamin E derivative or salt thereof

Examples of the vitamin E derivative include a tocopherol phosphoric acid ester and a salt thereof.
Examples of the salt of the tocopherol phosphoric acid ester include a salt of a tocopherol phosphoric acid ester with an inorganic base and a salt of a tocopherol phosphoric acid ester with an organic base.
Examples of the inorganic base and the organic base include the same ones as the above-mentioned inorganic bases and organic bases described for the ascorbic acid derivative.

·· Vitamin P derivative or salt thereof

Examples of the vitamin P derivative include methyl hesperidin obtained by methylating hesperidin. The methyl hesperidin is preferably a product solubilized in water.
As the methyl hesperidin, it is known that there are mainly a chalcone-type compound (chalcone-form methyl hesperidin) and a flavanone-type compound (flavanone-form methyl hesperidin); however, the methyl hesperidin is not limited to those.
Methyl hesperidin can be produced by a known method, for example, by dissolving hesperidin produced from citrus peels or the like in an aqueous solution of sodium hydroxide, allowing the corresponding amount of dimethyl sulfuric acid to act on the alkali solution, neutralizing the reaction liquid with sulfuric acid, performing extraction with n-butyl alcohol, distilling off the solvent, and then performing recrystallization from isopropyl alcohol.
As the hydroxy compound according to the present embodiment, among those described above, a hydroxy compound having a hydroxy equivalent (molecular weight of the hydroxy compound / number of hydroxy groups of the hydroxy compound) of preferably 10 or greater and 500 or less, more preferably 10 or greater and 300 or less, even more preferably 10 or greater and 100 or less, particularly preferably 30 or greater and 85 or less, and most preferably 50 or greater and 85 or less.
Among those described above, it is preferable that the hydroxy compound according to the present embodiment is a sugar, or a vitamin P derivative or a salt thereof.
More specifically, the hydroxy compound is preferably one or more compounds selected from the group consisting of mannitol, an oligosaccharide, a vitamin P derivative, and a salt of a vitamin P derivative; more preferably one or more compounds selected from the group consisting of mannitol, sucralose, α-cyclodextrin,β-cyclodextrin, an α-cyclodextrin derivative, a β-cyclodextrin derivative, and methyl hesperidin; even more preferably one or more compounds selected from the group consisting of D-mannitol, sucralose, α-cyclodextrin, or HP-β-cyclodextrin, and methyl hesperidin;

and particularly preferably sucralose or α-cyclodextrin.

Regarding the hydroxy compound used in the first step, one kind thereof may be used alone, or two or more kinds thereof may be used in combination.

The use amount of the hydroxy compound used in the first mixing step of the present embodiment is preferably 200 parts by mass or greater, more preferably 500 parts by mass or greater, and even more preferably 800 parts by mass or greater, with respect to 100 parts by mass of the fine particles.

In addition, the use amount of the hydroxy compound in the first mixing step of the present embodiment is preferably 2,500 parts by mass or less, more preferably 2,200 parts by mass or less, and even more preferably 2,000 parts by mass or less, with respect to 100 parts by mass of the fine particles.

For example, the use amount of the hydroxy compound in the first mixing step of the present embodiment is preferably 200 parts by mass or greater and 2,500 parts by mass or less, more preferably 500 parts by mass or greater and 2,200 parts by mass or less, and even more preferably 800 parts by mass or greater and 2,000 parts by mass or less, with respect to 100 parts by mass of the fine particles.

In a case where the use amount of the hydroxy compound in the first mixing step of the present embodiment is within the above-described preferred range, the surface of the hydroxy compound can be more uniformly coated with fine particles.

The average particle diameter D50 of the hydroxy compound used in the first mixing step of the present embodiment is preferably 0.2 μm or greater and 300 μm or less, more preferably 1 μm or greater and 250 μm or less, even more preferably 10 μm or greater and 200 μm or less, and particularly preferably 30 μm or greater and 150 μm or less, from the viewpoint of further obtaining the effects of the present invention.

The ratio between the average particle diameter D50 of the hydroxy compound and the average particle diameter D50 of the fine particles (average particle diameter D50 of the hydroxy compound / average particle diameter D50 of the fine particles) used in the first mixing step of the present embodiment is preferably 10 or greater and 10,000 or less, more preferably 50 or greater and 7,500 or less, even more preferably 100 or greater and 6,000 or less, and particularly preferably 1,000 or greater and 4,500 or less, from the viewpoint of further obtaining the effects of the present invention.

In a case where the liquid mixture includes two or more kinds of fine particles and two or more kinds of hydroxy compounds, the ratio between the average particle diameter D50 measured for all the hydroxy compounds and the average particle diameter D50 measured for all the fine particles may be within the above-described preferable range; however, it is acceptable when the ratio between the average particle diameter D50 of at least one hydroxy compound and the average particle diameter D50 of at least one fine particles is within the above-described preferable range.

The first mixing step in the method for producing a liquid mixture of the present embodiment may be specifically a chemical surface modification method of causing a chemical reaction to attach the fine particles to the hydroxy compound, or a physical surface modification method of applying physical energy to attach the fine particles to the hydroxy compound; however, a physical surface modification method is preferred.

Specifically, from the viewpoint of further dispersing the fine particles, a mechanical surface modification method (mechanochemical method) is more preferable as the physical surface modification method.

The mechanical surface modification method is a technique of repeatedly subjecting two or more kinds of particles having different particle diameters, compositions, and the like to compression, shearing, impact, and the like and thereby fixing fine particles (guest particles) or forming a film of fine particles (guest particles) on the surface of core particles (host particles).

The mixing apparatus for performing the first mixing step in the method for producing a liquid mixture of the present embodiment is not particularly limited, and any known mixing apparatus can be used.

In a case where the mechanical surface modification method is performed as the first mixing step, as an apparatus for performing the mechanical surface modification method, for example, a planetary ball mill (manufactured by Fritsch GmbH), a hybridization system (manufactured by Nara Machinery Co., Ltd.), a MECHANO FUSION (registered trademark) system (manufactured by Hosokawa Micron Corporation), NOBILTA (registered trademark) (manufactured by Hosokawa Micron Corporation), NANOCULAR (registered trademark) (manufactured by Hosokawa Micron Corporation), MECHANOMILL (registered trademark) (manufactured by Okada Seiko Co., Ltd.), THETA COMPOSER (registered trademark) (manufactured by Tokuju Co., Ltd.), NANOSONIC MILL (manufactured by Inoue Manufacturing, Inc.), KNEADER (manufactured by Inoue Manufacturing, Inc.), SUPERMASSCOLLOIDER (manufactured by Masuko Sangyo Co., Ltd.), NANO-MECH REACTOR (manufactured by Techno-Eye, Inc.), and CORNELL DESPA (manufactured by Asada Iron Works Co., Ltd.) can be used.

In the first mixing step, it is preferable that the mixing conditions are set such that the loading power on the fine particles and the hydroxy compound is preferably 1 W/g or greater, more preferably 3 W/g or greater, and even more preferably 5 W/g or greater.

Here, the power P (kW) can be determined by the following Equation (1).

$$\text{Power P (kW)} = (\text{Current} \times \text{voltage} \times \text{efficiency} \times \text{power factor}) / 1000 \quad \cdots (1)$$

The upper limit value of the loading power is not particularly limited and is set to the extent that the hydroxy compound is not excessively pulverized. For example, the upper limit value of the loading power may be 100 W/g or less, may be 50 W/g or less, or may be 10 W/g or less.

The mixing temperature for the first mixing step is not particularly limited as long as each component does not deteriorate, and the mixing temperature is, for example, 5°C to 100°C.

The mixing time for the first mixing step is not particularly limited as long as each component does not deteriorate, and the mixing time is, for example, 1 to 30 minutes.

<Second mixing step>

A second mixing step in the method for producing a liquid mixture of the present embodiment is a step of mixing the hydroxy compound coated with fine particles with a solvent, and obtaining a liquid mixture in which fine particles coated with a hydroxy compound are dispersed in a solvent.

In the first mixing step, the hydroxy compound is coated with the fine particles; however, in the second mixing step, most of the hydroxy compound is dissolved in the solvent, so that the host particles and the guest particles are reversed, and fine particles coated with the hydroxy compound are created.

Here, the "fine particles coated with a hydroxy compound" are fine particles whose surface is partially or entirely covered with a hydroxy compound.

The coating amount of the hydroxy compound with respect to the fine particles can be controlled by, for example, the use amount of the hydroxy compound with respect to 100 parts by mass of the fine particles in the above-mentioned first mixing step.

The average particle diameter D50 of the fine particles in the liquid mixture obtained in the second mixing step is preferably 410 nm or less, more preferably 5 nm or greater and 200 nm or less, even more preferably 30 nm or greater and 100 nm or less, particularly preferably 40 nm or greater and 95 nm or less, and most preferably 50 nm or greater and 90 nm or less.

<<Solvent>>

Examples of the solvent according to the present embodiment include water, an organic solvent, and a solvent mixture of those.

Examples of the organic solvent include an ester-based solvent, a ketone-based solvent, an ether-based solvent, an alcohol-based solvent, a nitrile-based solvent, an amide-based solvent, a sulfoxide-based solvent, a fluorine-based inert liquid, a hydrocarbon-based solvent, and a silicone-based solvent.

<<Ester-based solvent>>

The ester-based solvent is an organic solvent including (-C(=O)-O-) in the structure thereof.

Examples of the ester-based solvent include methyl acetate, ethyl acetate, propyl acetate, isopropyl acetate, butyl acetate, amyl acetate, isoamyl acetate, ethyl methoxyacetate, ethyl ethoxyacetate, 2-methoxybutyl acetate, 3-methoxybutyl acetate, 4-methoxybutyl acetate, 3-methoxy-3-methylbutyl acetate, 3-ethyl-3-methoxybutyl acetate, ethylene glycol monoethyl ether acetate, ethylene glycol monopropyl ether acetate, ethylene glycol monobutyl ether acetate, ethylene glycol monophenyl ether acetate, diethylene glycol monomethyl ether acetate, diethylene glycol monoethyl ether acetate, diethylene glycol monopropyl ether acetate, diethylene glycol monobutyl ether acetate, diethylene glycol monophenyl ether acetate, propylene glycol monomethyl ether acetate (PGMEA), propylene glycol monoethyl ether acetate, propylene glycol monopropyl ether acetate, 2-ethoxybutyl acetate, 4-ethoxybutyl acetate, 4-propoxybutyl acetate, 2-methoxypentyl acetate, 3-methoxypentyl acetate, 4-methoxypentyl acetate, 2-methyl-3-methoxypentyl acetate, 3-methyl-3-methoxypentyl acetate, 3-methyl-4-methoxypentyl acetate, 4-methyl-4-methoxypentyl acetate, propylene glycol diacetate, methyl formate, ethyl formate, propyl formate, butyl formate, ethyl lactate (EL), propyl lactate, butyl lactate, ethyl carbonate, propyl carbonate, butyl carbonate, methyl pyruvate, ethyl pyruvate, propyl pyruvate, butyl pyruvate, methyl acetoacetate, ethyl acetoacetate, methyl propionate, ethyl propionate, propyl propionate, isopropyl propionate, methyl 2-hydroxypropionate, ethyl 2-hydroxypropionate, methyl-3-methoxypropionate, ethyl-3-methoxypropionate, ethyl-3-ethoxypropionate, and propyl-3-methoxypropionate.

<<Ketone-based solvent>>

The ketone-based solvent is an organic solvent having a carbonyl group (ketone: -C(=O)-) other than an ester bond.

Examples of the ketone-based solvent include acetone, 1-hexanone, 2-hexanone, 4-heptanone, 2-heptanone (methyl amyl ketone), 1-octanone, 2-octanone, 1-nonanone, 2-nonanone, diisobutyl ketone, methyl ethyl ketone, methyl isobutyl ketone, acetyl acetone, acetonyl acetone, phenyl acetone, acetophenone, methyl naphthyl ketone, cyclohexanone (CHN), methyl cyclohexanone, ionone, isophorone, diacetonyl alcohol, diacetone alcohol, and acetyl carbinol.

<<Ether-based solvent>>

The ether-based solvent is an organic solvent having an ether bond (-O-) other than an ester bond. Examples of the ether-based solvent include alkylene glycol monoalkyl ethers such as propylene glycol monomethyl ether (PGME), ethylene glycol monomethyl ether, ethylene glycol monoethyl ether, ethylene glycol monopropyl ether, ethylene glycol monobutyl ether, ethylene glycol monohexyl ether, ethylene glycol monophenyl ether, ethylene glycol mono-2-ethyl butyl ether, propylene glycol monoethyl ether, propylene glycol monopropyl ether, and propylene glycol monobutyl ether; and polyhydric alcohol partial ethers, such as ether group-containing alkylene glycol monoalkyl ether compounds such as diethylene glycol monomethyl ether, diethylene glycol monoethyl ether, diethylene glycol monopropyl ether, diethylene glycol monobutyl ether, diethylene glycol monohexyl ether, dipropylene glycol monomethyl ether, dipropylene glycol monoethyl ether, and dipropylene glycol monopropyl ether.

<<Alcohol-based solvent>>

The alcohol-based solvent is an organic solvent including an alcoholic hydroxyl group in the structure thereof. The term "alcoholic hydroxyl group" means a hydroxyl group bonded to a carbon atom of an aliphatic hydrocarbon group.

In the present specification, the alcohol-based solvent is not included in the ester-based solvent, the ketone-based solvent, and the ether-based solvent.

Examples of the alcohol-based solvent include monoalcohols such as methanol, ethanol, n-propanol, isopropanol (IPA), n-butanol, sec-butanol, t-butanol, n-pentanol, 4-methyl-2-pentanol (methyl isobutyl carbinol), and 2-methylbutyl alcohol; and polyhydric alcohols such as ethylene glycol, diethylene glycol, propylene glycol, and dipropylene glycol.

<<Nitrile-based solvent>>

The nitrile-based solvent is an organic solvent including a nitrile group (-C=N) in the structure thereof.

Examples of the nitrile-based solvent include acetonitrile, propionitrile, valeronitrile, and butyronitrile.

<<Amide-based solvent>>

The amide-based solvent is an organic solvent including an amide group in the structure thereof.

Examples of the amide-based solvent include N,N-dimethylformamide, N-methylformamide, N,N-dimethylacetamide, N-methylacetamide, and N,N-diethylacetamide.

<<Sulfoxide-based solvent>>

The sulfoxide-based solvent is an organic solvent including a sulfinyl group (-S(=O)-) to which two alkyl groups are bonded, in the structure thereof.

Examples of the sulfoxide-based solvent include dimethyl sulfoxide.

<<Hydrocarbon-based solvent>>

Examples include hexane, heptane, octane, dodecane, cyclohexane, methylcyclohexane, isooctane, and hydrogenated triisobutylene.

<<Silicone-based solvent>>

Examples include octamethylcyclotetrasiloxane (D4), decamethylcyclopentasiloxane (D5), hexamethyldisiloxane, octamethyltrisiloxane, and polydimethylsiloxane (1cs, 6cs, and the like).

Among the above-described solvents, the solvent according to the present embodiment is preferably a solvent that appropriately dissolves the above-mentioned hydroxy compound, and specifically, it is more preferable that the solvent is a solvent including water or an alcohol-based solvent, while it is even more preferable to use water alone or an alcohol-based solvent alone.

Regarding the solvent of the present embodiment, one kind thereof may be used alone, or two or more kinds thereof may be used in combination.

<<Optional components>>

In the second mixing step of the present embodiment, optional components other than the hydroxy compound coated with fine particles obtained in the above-mentioned first mixing step and the above-described solvent may be added and mixed.

Specific examples of the optional components include a plasticizer, a high-molecular-weight dispersant; antioxidants such as a phenol-based antioxidant, a hindered amine-based antioxidant, a phosphorus-based antioxidant, a sulfur-based antioxidant, a benzotriazole-based antioxidant, a benzophenone-based antioxidant, a hydroxyamine-based antioxidant, a salicylic acid ester-based antioxidant, and a triazine-based antioxidant; and corrosion inhibitors such as benzotriazole or a derivative thereof, thiadiazole, and benzothiazole.

In the second mixing step of the present embodiment, from the viewpoint of further improving the dispersibility and dispersion stability of the fine particles, it is preferable to mix a high-molecular-weight dispersant in addition to the hydroxy compound coated with fine particles and the solvent.

<<High-molecular-weight dispersant>>

The high-molecular-weight dispersant is a surfactant structurally having a hydrophilic moiety and a lipophilic moiety and is a high-molecular-weight compound having a chain of monomers with a molecular weight of about 2,000 or greater.

There are also high-molecular-weight dispersants corresponding to the above-mentioned hydroxy compound.

That is, in the second mixing step of the present embodiment, the hydroxy compound in the hydroxy compound coated with fine particles and the high-molecular-weight dispersant that is added separately may be the same or different from each other. Above all, it is preferable that the hydroxy compound in the hydroxy compound coated with fine particles is different from the high-molecular-weight dispersant that is added separately, and it is more preferable that the hydroxy compound is a compound that does not correspond to the high-molecular-weight dispersant.

Specific examples of the high-molecular-weight dispersant include an anionic high-molecular-weight dispersant, a nonionic high-molecular-weight dispersant, and a cationic high-molecular-weight dispersant.

· Anionic high-molecular-weight dispersant

More specific examples of the anionic high-molecular-weight dispersant include a styrene-maleic anhydride copolymer; a formalin conjugate of a naphthalene sulfonic acid salt; a polyacrylic acid salt; carboxymethyl cellulose; an olefin-maleic anhydride copolymer; a polystyrene sulfonic acid salt; an acrylamide-acrylic acid copolymer; and sodium alginate (natural).

· Nonionic high-molecular-weight dispersant

More specific examples of the nonionic high-molecular-weight dispersant include a polyvinyl alcohol-acrylic acid-methyl methacrylate copolymer; hydroxypropyl cellulose; hydroxypropyl methyl cellulose; polyvinyl alcohol; polyoxyethylene alkyl ether; polyalkylene polyamine; polyacrylamide; polyoxypropylene-polyoxyethylene block; and polymer starch (natural).

· Cationic high-molecular-weight dispersant

More specific examples of the cationic high-molecular-weight dispersant include polyethyleneimine; an aminoalkyl (meth)acrylate copolymer; polyvinyl imidazoline; and satokinsan (natural).

Among those described above, the high-molecular-weight dispersant is preferably a nonionic high-molecular-weight dispersant from the viewpoint of further improving the dispersibility and dispersion stability of the fine particles. Specifically, it is more preferable that the high-molecular-weight dispersant is one or more nonionic high-molecular-weight dispersants selected from the group consisting of a polyvinyl alcohol-acrylic acid-methyl methacrylate copolymer, hydroxypropyl cellulose, and hydroxypropyl methyl cellulose.

Regarding the high-molecular-weight dispersant according to the present embodiment, one kind thereof may be used alone, or two or more kinds thereof may be used in combination.

The use amount of the high-molecular-weight dispersant in the second mixing step of the present embodiment is preferably 200 parts by mass or greater, more preferably 300 parts by mass or greater, and even more preferably 400 parts by mass or greater, with respect to 100 parts by mass of the fine particles.

In addition, the use amount of the high-molecular-weight dispersant in the second mixing step of the present embodiment is preferably 2,000 parts by mass or less, more preferably 1,500 parts by mass or less, and even more preferably 1,000 parts by mass or less, with respect to 100 parts by mass of the fine particles.

For example, the use amount of the high-molecular-weight dispersant in the second mixing step of the present embodiment is preferably 200 parts by mass or greater and 2,000 parts by mass or less, more preferably 300 parts by mass or greater and 1,500 parts by mass or less, and even more preferably 400 parts by mass or greater and 1,000 parts by mass or less, with respect to 100 parts by mass of the fine particles.

In a case where the use amount of the high-molecular-weight dispersant in the second mixing step of the present embodiment is within the above-described preferred range, the dispersibility and dispersion stability of the fine particles can be further improved.

With regard to the second mixing step of the present embodiment, in a case where optional components are blended, the blending order of each of the components is not particularly limited. Mixing may be carried out after all the components are added, mixing may be carried out while sequentially adding some of the components, or mixing may be carried out while sequentially adding all of the components.

The second mixing step in the method for producing a liquid mixture of the present embodiment is not particularly limited as long as the second mixing step is a general method of mixing a solid and a liquid.

Specifically, the method may be appropriately selected from known methods such as a method of mixing by rotating a stirring bar, a stirring blade, or the like; a method of mixing by using a mixer, a three-roll, a kneader, a bead mill, or the like; and a method of mixing by applying ultrasonic waves.

A mixing apparatus for performing the second mixing step in the method for producing a liquid mixture of the present embodiment is not particularly limited, and a known mixing apparatus can be used.

Specifically, an ultrasonic homogenizer (trade name: UD-200, manufactured by Tomy Seiko Co., Ltd.) or the like can be used.

The mixing temperature for the second mixing step is not particularly limited as long as each component does not deteriorate, and the mixing temperature is, for example, 5°C to 100°C.

The mixing time for the second mixing step is not particularly limited as long as each component does not deteriorate, and the mixing time is, for example, 1 minute to 1 hour.

The method for producing a liquid mixture of the present embodiment as described above includes a first mixing step of mixing fine particles and a hydroxy compound to obtain a hydroxy compound coated with fine particles, and a second mixing step of mixing the hydroxy compound coated with fine particles with a solvent to obtain a liquid mixture in which fine particles coated with a hydroxy compound are dispersed in a solvent. As a result, the dispersibility and dispersion stability of the fine particles can be dramatically improved as compared with a conventional method of simply mixing each raw material to produce a liquid mixture.

This is speculated to be because the fine particles resulting from crushing of aggregation uniformly attach to the surface of the hydroxy compound through the first mixing step, and then by mixing the hydroxy compound coated with the fine particles with the solvent in the second mixing step, the hydroxy compound is dissolved in a state in which aggregation of the fine particles has been crushed.

(Liquid mixture)

The liquid mixture of the present embodiment is a liquid mixture in which fine particles coated with a hydroxy compound are dispersed in a solvent, and the hydroxy compound is one or more compounds selected from the group consisting of D-mannitol, $\alpha$-cyclodextrin, HP-$\beta$-cyclodextrin, methyl hesperidin, and sucralose.

The liquid mixture of the present embodiment is typically a liquid mixture produced by the above-mentioned method for producing a liquid mixture.

Examples of the hydroxy compound, the fine particles, the solvent, and the optional components that are added as necessary in the liquid mixture of the present embodiment include those similar to the hydroxy compound, the fine particles, the solvent, and the optional components for the above-mentioned method for producing a liquid mixture.

The content of the hydroxy compound in the liquid mixture of the present embodiment is preferably 200 parts by mass or greater and 2,000 parts by mass or less, more preferably 500 parts by mass or greater and 1,500 parts by mass or less, and even more preferably 800 parts by mass or greater and 1,200 parts by mass or less, with respect to 100 parts by mass of the fine particles.

Regarding the average particle diameter D50 of the fine particles in the liquid mixture of the present embodiment,

from the viewpoint of further obtaining the effects of the present invention, the average particle diameter D50 is preferably 410 nm or less, more preferably 5 nm or greater and 200 nm or less, even more preferably 30 nm or greater and 100 nm or less, particularly preferably 40 nm or greater and 95 nm or less, and most preferably 50 nm or greater and 90 nm or less.

The average particle diameter D50 of the fine particles in the liquid mixture of the present embodiment is within the above-described preferable range, and the span value of the fine particles in the liquid mixture of the present embodiment is preferably 58 or less, more preferably 10 or less, even more preferably 9 or less, still more preferably 8 or less, even more preferably 6.5 or less, and most preferably 1 or less.

Here, the "span value" is an index indicating the sharpness of the particle size distribution and can be determined by (D90 - D10) / D50. A smaller span value means that the particle size distribution is sharper.

Here, the term D10 means the particle diameter at a cumulative percentage of 10% from the smaller diameter side on the basis of a cumulative volume distribution diagram of the particle size distribution of the fine particles, and the term D90 means the particle diameter at a cumulative percentage of 90% from the smaller diameter side on the basis of a cumulative volume distribution diagram of the particle size distribution of the fine particles. Both the D10 and the D90 can all be measured in the same manner as in the case of the above-mentioned D50.

In a case where two or more kinds of fine particles are included in the liquid mixture, it is desirable that for all the fine particles, the average particle diameter D50 is within the above-described preferable range, while the span value is within the above-described preferable range; however, it is desirable that the average particle diameter D50 of at least one kind of the fine particles is within the above-described preferable range, while the span value is within the above-described preferable range.

The liquid mixture of the present embodiment is used as a coating agent, a paint, or a granulating liquid.

In addition, the liquid mixture can also be used as a cream (semi-solid preparation) as a pharmaceutical or cosmetic product, by further adding a paste oil, a wax, or the like.

Among the above, the liquid mixture of the present embodiment is particularly useful as a coating agent for coating tablets or the like.

In the liquid mixture of the present embodiment as described above, since the fine particles are coated with the hydroxy compound, the fine particles are satisfactorily dispersed in the solvent.

Therefore, for example, in a case where the liquid mixture of the present embodiment is used as a coating agent, the fine particles can be uniformly attached to the surface of an object to be coated.

In addition, the liquid mixture of one embodiment is a liquid mixture containing a hydroxy compound and fine particles, in which the average particle diameter D50 of the fine particles is 200 nm or less, and the span value of the fine particles is 9 or less.

Preferred aspects of the hydroxy compound and the fine particles in the liquid mixture of the present embodiment are as described above.

The average particle diameter D50 of the fine particles in the liquid mixture of the present embodiment is 200 nm or less, preferably 5 nm or greater and 200 nm or less, more preferably 30 nm or greater and 100 nm or less, even more preferably 40 nm or greater and 95 nm or less, and particularly preferably 50 nm or greater and 90 nm or less.

The span value of the fine particles in the liquid mixture of the present embodiment is 9 or less, preferably 8 or less, more preferably 6.5 or less, and even more preferably 1 or less.

(Solid composition)

Specific examples of the solid composition of the present embodiment include a solid composition coated with the above-mentioned liquid mixture and a solid composition created using the above-mentioned liquid mixture.

More specific examples of the solid composition include a solid pharmaceutical composition, a solid cosmetic, and a solid food.

<<Solid composition coated with liquid mixture>>

In the present specification, the phrase "solid composition coated with the liquid mixture" specifically means a solid composition having a coating layer formed using the liquid mixture on the surface. That is, the "solid composition coated with the liquid mixture" is not simply limited to a product obtained by attaching the liquid mixture to the solid composition, drying the solvent, and forming a coating layer on the surface of the solid composition, and for example, a gelling agent may be further blended into the liquid mixture, a film is formed, and the solid composition may be coated with the film. In addition, an aspect in which a capsule is created from the liquid mixture by a rotary-type method, a seamless-type method, or the like, and the solid composition is coated with the capsule, may also be employed.

Typical examples of the solid composition coated with the liquid mixture include a solid pharmaceutical composition coated with the liquid mixture and a solid cosmetic coated with the liquid mixture.

· Solid pharmaceutical composition coated with liquid mixture

The dosage form of the solid pharmaceutical composition coated with the liquid mixture is not particularly limited, and examples thereof include a tablet, a pill, a powder, and a granular preparation.
The solid pharmaceutical compositions contain a drug and a pharmaceutically acceptable carrier.
Specific examples of the drug include amlodipine, evastin, selegiline, brotizolam, ramosetron, midodrin, montelukast, azulene sulfonic acid, etizolam, bromperidol, mecobalamin, alpha-calcidol, bromocriptine, pramipexole, rosuvastatin, silodosin, nifedipine, and pharmacologically acceptable salts or solvates thereof.
Specific examples of the pharmacologically acceptable salt of the above-described drugs include amlodipine besylate.
The pharmaceutically acceptable carrier is not particularly limited, and carriers generally used for pharmaceutical products can be used. For example, those general raw materials described in the Japanese Pharmacopoeia, the Japanese Pharmaceutical Codex, Japanese Pharmaceutical Excipients 2018 (Yakuji Nippo, Ltd., 2018), the Japanese Pharmaceutical Excipients Directory 2021 (edited by International Pharmaceutical Excipients Council Japan, Yakuji Nippo, Ltd., 2021), Handbook of Pharmaceutical Excipients, 7th edition (Pharmaceutical Press, 2012), and the like can be used.
Regarding the pharmaceutically acceptable carrier, one kind thereof may be used alone, or two or more kinds thereof may be used in combination.
More specific examples of a pharmaceutically acceptable carrier include an excipient, a disintegrating agent, a binder, a flavoring agent, a coloring agent, and a lubricating agent.
Specific examples of the excipient include starches such as cornstarch and potato starch, microcrystalline cellulose, talc, and mannitol.
Specific examples of the disintegrating agent include a partially pregelatinized starch, carmellose calcium, crospovidone, low-substituted hydroxypropyl cellulose, croscarmellose sodium, and carboxymethyl starch sodium.
Specific examples of the binder include polyvinylpyrrolidone, hydroxypropyl methyl cellulose, hydroxypropyl cellulose, gum arabic powder, gelatin, pullulan, carmellose sodium, ethyl cellulose, and an aminoalkyl methacrylate copolymer.
Specific examples of the corrigent include aspartame, sucralose, saccharin sodium, glycyrrhizin dipotassium, stevia, thaumatin, and citric acid.
Specific examples of the flavoring agent include menthol, peppermint micron, lemon, lemon lime, orange, peppermint oil, and various flavors.
Specific examples of the coloring agent include iron oxide, a tar-based pigment, turmeric extract, caramel, carotene liquid, β-carotene, copper chlorophyll, and riboflavin.
Specific examples of the lubricating agent include magnesium stearate, polyethylene glycol, liquid paraffin, silicones, and surfactants such as long-chain fatty acid esters; and waxes such as beeswax, carnauba, and paraffin.

· Solid cosmetic coated with liquid mixture

The form of the solid cosmetic coated with the liquid mixture is not particularly limited, and examples thereof include base makeup cosmetics such as foundation and makeup base; and point makeup cosmetics such as eye shadow, lipstick, lip gloss, and blusher.
The components contained in the solid cosmetic are not particularly limited, and general raw materials for cosmetic products can be used. For example, general raw materials described in the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervisorily edited by the Examination Division of the Pharmaceutical Affairs Bureau, Yakuji Nippo, Ltd., 1993); Supplement to the Japanese Cosmetic Ingredients Codex (supervisorily edited by the Examination Division of the Pharmaceutical Affairs Bureau, Yakuji Nippo, Ltd., 1993); the Comprehensive Licensing Standards of Cosmetics by Category (supervisorily edited by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like can be used.
Regarding the solid composition coated with the liquid mixture, the solid composition can be coated with the above-described liquid mixture by a known method. For example, a spin coating method, an immersion method (dipping method), or a spraying method can be used.
The spin coating method is a method of rotating the solid composition by using a spin coater or the like and dripping or spraying the liquid mixture onto the rotating solid composition.
The immersion method (dipping method) is a method of immersing the solid composition in the liquid mixture.
The spraying method is a method of conveying the solid composition in a predetermined direction and spraying

the liquid mixture to the space.

In addition, the liquid mixture may be further blended with a gelling agent such as gelatin, agar, carrageenan, pectin, locust bean gum, xanthan gum, guar gum, gum arabic, taraya gum, karaya gum, alginic acid, tara gum, or starch, a film may be created from the liquid mixture by a known method, and the solid composition may be coated with the film.

In addition, a liquid mixture in which a gelling agent is blended may be used, and the liquid mixture may be produced into a soft capsule by a known rotary-type or seamless-type method to coat the solid composition.

Regarding an apparatus for producing a solid composition coated with a liquid mixture, for example, a fluidized bed granulator dryer (trade name: MP-01, manufactured by Powrex Corporation) or a film coating apparatus (trade name: HCT-LABO, Freund Corporation) can be used.

<<Solid composition created by liquid mixture>>

Specific examples of the solid composition created using the liquid mixture include a resin film.

The resin film contains the liquid mixture of the above-described embodiment, a thermoplastic resin, and other components as necessary.

Examples of the thermoplastic resin include polyolefin resins such as polyethylene, polypropylene, and polybutadiene; polyester-based resins such as polyethylene terephthalate, polyethylene naphthalate, polybutylene terephthalate, and polytrimethylene terephthalate; polyamide-based resins such as polyamide 6 (nylon 6), polyamide 66 (nylon 66), polyamide 11 (nylon 11), polyamide 12 (nylon 12), polyamide 46 (nylon 46), polyamide 610 (nylon 610), polytetramethylene terephthalamide (nylon 4T), polyhexamethylene terephthalamide (nylon 6T), poly-meta-xylylene adipamide (nylon MXD6), polynonamethylene terephthalamide (nylon 9T), and polydecamethylene terephthalamide (nylon 10T); vinyl-based resins such as vinyl chloride, vinylidene chloride vinyl acetate, and polyvinyl alcohol; polystyrene-based resins such as polystyrene, an acrylonitrile-styrene resin (AS resin), and an acrylonitrile-butadienestyrene resin (ABS resin); polysulfone-based resins such as modified polysulfone, polyethersulfone, polysulfone, and polyphenylsulfone; polyphenylene sulfides such as linear polyphenylene sulfide, crosslinked polyphenylene sulfide, and semi-crosslinked polyphenylene sulfide; polyether ketones such as polyether ketone, polyether ether ketone, and polyether ketone ketone; polycarbonates; polyphenylene ether; and polyimide-based resins such as thermoplastic polyimide, polyamide-imide, and polyetherimide.

Examples of the other components include a conductivity imparting agent, an ultraviolet absorber, an antioxidant, an antibacterial agent, an insect repellent, a deodorant, a coloring inhibitor, a thermal stabilizer, a mold release agent, an antistatic agent, a plasticizer, a lubricating agent, a coloring agent, a pigment, a dye, a foaming agent, an antifoaming agent, a viscosity modifier, and a surfactant.

The resin film can be produced by, for example, melt-kneading the liquid mixture of the above-described embodiment, a thermoplastic resin, and other components as necessary by using a melt extruder, extruding the resultant through a discharge port of an extrusion die, and subjecting the extrusion product to a cooling step.

In addition, the method for producing the resin film is not particularly limited, and known methods such as a melt extrusion molding method such as an inflation method or a T-die method; a solution flow casting method; and a calender method can be used.

The solid composition of the present embodiment as described above is coated with the above-mentioned liquid mixture.

Accordingly, for example, in a case where the solid composition is a solid pharmaceutical composition, and the fine particles contained therein have a light scattering effect or the like, since the fine particles are uniformly attached to the surface of the solid pharmaceutical composition, it is difficult for light to reach the drug contained in the solid pharmaceutical composition due to the light scattering and absorption effect of the fine particles, and thus the light stability of the solid pharmaceutical composition can be improved.

[Examples]

Next, the present invention will be described in more detail by way of Examples; however, the present invention is not limited to the following Examples.

[Production 1 of liquid mixture]

(Examples 1 to 5)

First mixing step:

0.1 g of ultramicronized iron sesquioxide (trade name: Ultramicronized Iron sesquioxide, manufactured by

IoLiTec Ionic Liquids Technologies GmbH, average particle diameter D50: 20 to 40 nm) and 0.9 g of each of the hydroxy compounds shown in the following Table 1 were subjected to a dry compositing treatment using a dry compositing apparatus (trade name: NOBILTA (registered trademark) Mini, manufactured by Hosokawa Micron Corporation) to obtain a hydroxy compound coated with ultramicronized iron sesquioxide (composite powder A).

Second mixing step:

1 g of the composite powder A, 1 g of a high-molecular-weight dispersant A (trade name: POVACOAT, manufactured by Daido Chemical Corporation, compound name: polyvinyl alcohol-acrylic acid-methyl methacrylate copolymer), and 19 g of water were mixed using an ultrasonic homogenizer (trade name: UD-200, manufactured by Tomy Seiko Co., Ltd.) to obtain each of liquid mixtures of Examples 1 to 5, in which ultramicronized iron sesquioxide coated with a hydroxy compound was dispersed in water.

(Comparative Example 1)

0.1 g of ultramicronized iron sesquioxide, 1 g of the high-molecular-weight dispersant A, and 19 g of water were mixed using an ultrasonic homogenizer to obtain a liquid mixture of Comparative Example 1.

(Comparative Example 2)

0.1 g of ultramicronized iron sesquioxide, 0.9 g of D-mannitol (trade name: PEARLITOL 50C, manufactured by Roquette Freres SA), 1 g of the high-molecular-weight dispersant A, and 19 g of water were mixed using an ultrasonic homogenizer to obtain a liquid mixture of Comparative Example 2.

(Comparative Examples 3 to 5)

Liquid mixtures of Comparative Examples 3 to 5 were obtained by a method similar to the method for producing the liquid mixtures of Examples 1 to 5, except that with regard to the production of the above-mentioned liquid mixtures of Examples 1 to 5, each of the hydroxy compounds shown in the following Table 1 was changed to each of the compounds having no hydroxy group shown in the following Table 2.

[Measurement of particle diameter of fine particles]

The average particle diameters D10, D50, and D90 of the ultramicronized iron sesquioxide in the liquid mixture of each Example were measured with a laser diffraction-type particle size distribution meter (trade name: LMS-2000, manufactured by Seishin Enterprise Co., Ltd.). The results are shown as "D50" in Tables 1, 2, and 5 to 7. In addition, the "span value" that can be determined by (D90 - D10) / D50 was also calculated, and the results are shown in Tables 1, 2, and 5 to 7.

[Table 1]

| | | Example 1 | Example 2 | Example 3 | Example 4 | Example 5 |
|---|---|---|---|---|---|---|
| Fine particles | Ultramicronized iron sesquioxide | 0.1 | 0.1 | 0.1 | 0.1 | 0.1 |
| Hydroxy compound | D-mannitol | 0.9 | - | - | - | - |
| | $\alpha$-CyD | - | 0.9 | - | - | - |
| | Methyl hesperidin | - | - | 0.9 | - | - |
| | HP-$\beta$-cyD | - | - | - | 0.9 | - |
| | Sucralose | - | - | - | - | 0.9 |
| High-molecular-weight dispersant | Povacoat | 1 | 1 | 1 | 1 | 1 |
| Solvent | Water | 19 | 19 | 19 | 19 | 19 |

(continued)

| | | | Example 1 | Example 2 | Example 3 | Example 4 | Example 5 |
|---|---|---|---|---|---|---|---|
| Evaluation of particle diameter | D50 | [nm] | 91 | 76 | 86 | 88 | 94 |
| | Span value | | 6.48 | 0.89 | 6.36 | 6.44 | 7.15 |

[Table 2]

| | | | Comparative Example 1 | Comparative Example 2 | Comparative Example 3 | Comparative Example 4 | Comparative Example 5 |
|---|---|---|---|---|---|---|---|
| Fine particles | Ultramicronized iron sesquioxide | | 0.1 | 0.1 | 0.1 | 0.1 | 0.1 |
| Compound | D-mannitol | | - | 0.9 | - | - | - |
| | Acesulfame potassium | | - | - | 0.9 | - | - |
| | Sodium lauryl sulfate | | - | - | - | 0.9 | - |
| | Sodium triphosphate | | - | - | - | - | 0.9 |
| High-molecular-weight dispersant | Povacoat | | 1 | 1 | 1 | 1 | 1 |
| Solvent | Water | | 19 | 19 | 19 | 19 | 19 |
| Evaluation of particle diameter | D50 | [nm] | 1357 | 1344 | 130 | 604 | 452 |
| | Span value | | 5.55 | 5.30 | 59.0 | 2.67 | 4.97 |

The terms in Tables 1 and 2 have the following meanings. The numerical values in the tables denote the blending amounts (g).

Ultramicronized iron sesquioxide (trade name: Ultramicronized Iron Sesquioxide, manufactured by IoLiTec Ionic Liquids Technologies GmbH, average particle diameter D50: 20 to 40 nm).

D-mannitol: D-mannitol (trade name: PEARLITOL 50C, manufactured byRoquette Freres SA, average particle diameter D50: 38 μm).

α-CyD: α-Cyclodextrin (trade name: CELDEX A-100, manufactured by Nihon Shokuhin Kako Co., Ltd., average particle diameter D50: 36 μm).

Methyl hesperidin: Methyl hesperidin (trade name: Methyl Hesperidin, manufactured by Alps Foods Co., Ltd., average particle diameter D50: 30.3 μm).

HP-β-CyD: Hydroxypropylated β-cyclodextrin (trade name: CELDEX HP-β-CD, manufactured by Nihon Shokuhin Kako Co., Ltd., average particle diameter D50: 47 μm).

Sucralose: (trade name: SU-600, manufactured by Tsuruya Chemical Industries, Ltd., average particle diameter D50: 57 μm).

Acesulfame potassium: (trade name: SUNETT, manufactured by Mitsubishi Corporation Life Sciences, Ltd., average particle diameter D50: 36 μm).

Sodium lauryl sulfate: (trade name: SLS, manufactured by Nikko Chemicals Co., Ltd., average particle diameter D50: 72 μm).

Sodium triphosphate: (trade name: sodium tripolyphosphate, manufactured by Taihei Chemical Industrial Co., Ltd., average particle diameter D50: 30 μm).

Povacoat: (trade name: POVACOAT, manufactured by Daido Chemical Corporation, compound name: polyvinyl alcohol-acrylic acid-methyl methacrylate copolymer).

As shown in Tables 1 and 2, it could be verified that in the liquid mixtures of the Examples, the average particle diameter D50 and the span value of the ultramicronized iron sesquioxide were small as compared to the liquid mixtures of the Comparative Examples, and the ultramicronized iron sesquioxide was satisfactorily dispersed.

Since the liquid mixtures of Comparative Examples 1 and 2 were obtained by simply mixing each of the raw materials with an ultrasonic homogenizer, the aggregation of the ultramicronized iron sesquioxide could not be crushed, and the average particle diameter D50 of the ultramicronized iron sesquioxide had a large value.

Since the liquid mixture of Comparative Example 3 did not contain a hydroxy compound, the average particle diameter D50 of the ultramicronized iron sesquioxide could be reduced; however, since the span value was large, the particle size distribution was broader, and the dispersibility of the ultramicronized iron sesquioxide was inferior.

In Comparative Examples 4 and 5, since a hydroxy compound was not contained, the aggregation of the ultramicronized iron sesquioxide could not be crushed, and the average particle diameter D50 of the ultramicronized iron sesquioxide had a large value.

[Production 1 of solid pharmaceutical composition]

(Examples 1a to 1c)

Using the liquid mixture of Example 1, uncoated tablets having the formulations shown in Table 3 were coated by using a film coating apparatus (trade name: HCT-LABO, manufactured by Freund Corporation).

A solid pharmaceutical composition of Example 1a, in which the weight of the coating film was 0.54 mg, a solid pharmaceutical composition of Example 1b, in which the weight of the coating film was 2 mg, and a solid pharmaceutical composition of Example 1c, in which the weight of the coating film was 5.4 mg, were each obtained.

(Comparative Examples 2a to 2c)

Using the liquid mixture of Comparative Example 2, uncoated tablets having the formulations shown in Table 3 were coated by using the above-described film coating apparatus.

A solid pharmaceutical composition of Comparative Example 2a, in which the weight of the coating film was 0.54 mg, a solid pharmaceutical composition of Comparative Example 2b, in which the weight of the coating film was 2 mg, and a solid pharmaceutical composition of Comparative Example 2c, in which the weight of the coating film was 5.4 mg, were each obtained.

[Table 3]

| Configuration | Component | Blending amount (mg) | |
|---|---|---|---|
| Active granules | Amlodipine besylate | 6.93 | 6.93 |
| | D-mannitol | 31.87 | 31.87 |
| | Ethyl cellulose | 12 | 12 |
| | Talc | 9 | 9 |
| | Yellow iron sesquioxide | 0.2 | 0.2 |
| Active granules total | | 60 | 60 |
| Fast disintegrating granules | D-Mannitol | 93.4 | 93.4 |
| | Ethyl cellulose | 5 | 5 |
| | Cornstarch | 32.4 | 32.4 |
| | Crospovidone | 3.6 | 3.6 |
| | Yellow iron sesquioxide | 0.2 | 0.2 |
| Fast disintegrating granules total | | 134.6 | 134.6 |
| l-menthol | D-mannitol | 1.7 | 1.7 |
| Triturated powder | l-menthol | 0.5 | 0.5 |
| l-menthol triturated powder total | | 2.2 | 2.2 |
| Tablet | Light anhydrous silicic acid | 0.5 | 0.5 |
| | Aspartame | 5.3 | 5.3 |
| | Peppermint micron | 1 | 1 |
| | Magnesium stearate | 1.4 | 1.4 |
| Total | | 205 | 205 |

[Evaluation 1 of light stability]

The light stability of the solid pharmaceutical composition of each Example was evaluated by the following method, and the results are shown in FIG. 1.

The solid pharmaceutical composition of each Example was placed in a light stability apparatus and exposed to light with a total illuminance of 1.2 million lux hr under the conditions of 25°C by using a xenon lamp as a light source, and then the solid pharmaceutical composition of each Example was taken out of the testing apparatus. Then, a liquid mixture of an aqueous solution of potassium dihydrogen phosphate (4.1 → 1,000) and acetonitrile (volume ratio: 4:1) was added to the solid pharmaceutical composition of each Example to dissolve the contents, and thereby each sample solution was obtained.

An analogous substance (degradation product I) represented by the following Chemical Formula (1) in each sample solution was measured by an HPLC method.

[Chemical Formula 1]

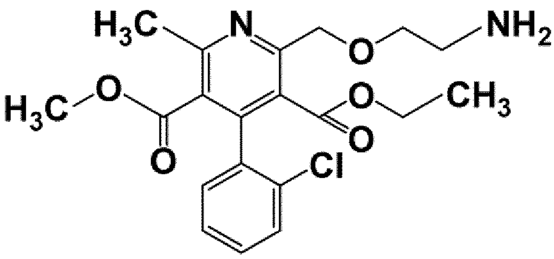

・・・（1）

The conditions for the HPLC method are as follows.

<<Measurement conditions>>

Detector: Ultraviolet absorption photometer (measurement wavelength: 237 nm)
Column: A column obtained by packing 3-μm octylsilylated silica gel for liquid chromatography into a stainless-steel tube having an inner diameter of 4.6 mm and a length of 15 cm.
Mobile phase flow rate: 1.0 mL per minute
Sample injection amount: 10 μL
Mobile phase: Composed of solutions A and B
Solution A: Aqueous solution of potassium dihydrogen phosphate (4.1 → 1000)
Solution B: Acetonitrile mixture
Feeding of mobile phase: Concentration gradient was controlled by changing the mixing ratio of the solution A and the solution B as shown in Table 4.

[Table 4]

| Time after injection (min) | Mobile phase A (vol%) | Mobile phase B (vol%) |
|---|---|---|
| 0 to 15 | 80 → 50 | 20 → 50 |
| 15 to 20 | 50 → 20 | 50 → 80 |
| 20 to 25 | 20 | 80 |
| 25 to 26 | 20 → 80 | 80 → 20 |
| 26 to 30 | 80 | 20 |

As shown in FIG. 1, it could be verified that the solid pharmaceutical compositions of Examples had a smaller amount of the degradation product I and satisfactory light stability, as compared with the solid pharmaceutical compositions of Comparative Examples.

[Production 2 of solid pharmaceutical composition]

(Example 2a)

Using the liquid mixture of Example 2, uncoated tablets having the formulations shown in Table 3 were coated by using a film coating apparatus (trade name: HCT-LABO, manufactured by Freund Corporation).
Thereby, a solid pharmaceutical composition of Example 2a, in which the weight of the coating film was 2 mg, was obtained.

(Comparative Example 6a)

0.1 g of iron sesquioxide (trade name: iron sesquioxide, manufactured by Kishi Kasei Co., Ltd., average particle diameter D50: 460 nm), 0.9 g of α-cyclodextrin (trade name: CELDEX A-100, manufactured by Nihon Shokuhin Kako Co., Ltd., average particle diameter D50: 36 μm), 1 g of the high-molecular-weight dispersant A, and 19 g of water were mixed using an ultrasonic homogenizer to obtain a liquid mixture of Comparative Example 6. Using the obtained liquid mixture, uncoated tablets having the formulation shown in Table 3 were coated by using a film coating apparatus (trade name: HCT-LABO, manufactured by Freund Corporation).
A solid pharmaceutical composition of Comparative Example 6a, in which the weight of the coating film was 2 mg, was obtained.

[Evaluation 2 of light stability]

An analogous substance (degradation product I) was measured by an HPLC method in the same manner as in [Evaluation 1 of light stability] described above, and the light stability of the above-mentioned solid pharmaceutical

compositions of Example 2a and Comparative Example 6a was evaluated.

In addition, not only the evaluation after the exposure to light with a total illuminance of 1.2 million lux·hr in the same manner as in [Evaluation 1 of light stability], but also the evaluation after exposure to light with a total illuminance of 300,000 lux·hr and 600,000 lux hr were performed.

The results are shown in FIG. 2.

As shown in FIG. 2, it could be verified that the solid pharmaceutical compositions of Examples had a smaller amount of the degradation product I and satisfactory light stability, as compared with the solid pharmaceutical compositions of Comparative Examples.

As described above, according to the method for producing a liquid mixture of the Examples, it was verified that a liquid mixture in which fine particles were present with satisfactory dispersibility and dispersion stability, could be obtained. In addition, it could be verified that a solid pharmaceutical composition coated with the liquid mixture had satisfactory light stability.

[Production 2 of liquid mixture]

(Examples 6 to 8)

Liquid mixtures of Examples 6 to 8 were obtained in the same manner as in the Examples of [Production 1 of liquid mixture], except that with regard to the above-described [Production 1 of liquid mixture], the high-molecular-weight dispersant A used in the second mixing step was changed to the high-molecular-weight dispersant shown in Table 5.

[Table 5]

| | | | Example 6 | Example 7 | Example 8 |
|---|---|---|---|---|---|
| Fine particles | Ultramicronized iron sesquioxide | | 0.1 | 0.1 | 0.1 |
| Hydroxy compound | α-CyD | | 0.9 | 0.9 | - |
| | Sucralose | | - | - | 0.9 |
| High-molecular-weight dispersant | HPC-SSL | | 1 | - | - |
| | HPMC | | - | 1 | - |
| | PVP K25 | | - | - | 1 |
| Solvent | Water | | 19 | 19 | - |
| | Ethanol | | - | - | 19 |
| Evaluation of particle diameter | D50 | [nm] | 72 | 75 | 82 |
| | Span value | | 0.88 | 0.94 | 0.86 |

The terms in Table 5 have the following meanings. The numerical values in the tables denote the blending amounts (g).

Ultramicronized iron sesquioxide (trade name: Ultramicronized Iron Sesquioxide, manufactured by IoLiTec Ionic Liquids Technologies GmbH, average particle diameter D50: 20 to 40 nm).

α-CyD: α-Cyclodextrin (trade name: CELDEX A-100, manufactured by Nihon Shokuhin Kako Co., Ltd., average particle diameter D50: 36 μm).

Sucralose: (trade name: J-600, manufactured by Tsuruya Chemical Industries, Ltd., average particle diameter D50: 134 μm).

HPC-SSL: Hydroxypropyl cellulose (trade name: NISSO HPC-SSL, manufactured by Nippon Soda Co., Ltd.).

HPMC: Hydroxypropyl methyl cellulose (trade name: TC-5R, manufactured by Shin-Etsu Chemical Co., Ltd.)

PVP K25: Polyvinylpyrrolidone (trade name: Kollidon 25, manufactured by BASF SE).

As shown in Tables 1 and 5, from a comparison between the liquid mixture of Example 2 and the liquid mixtures of Examples 6 and 7, and a comparison between the liquid mixture of Example 5 and the liquid mixture of Example 8, even when using any of the high-molecular-weight dispersants, the average particle diameter D50 and the span value of the ultramicronized iron sesquioxide could be reduced, and the ultramicronized iron sesquioxide could satisfactorily be dispersed.

[Production 3 of liquid mixture]

(Example 9)

A liquid mixture of Example 9 was produced by a production method similar to that of the Examples of [Production 1 of liquid mixture], except that with regard to the above-described Examples of [Production 1 of liquid mixture], the hydroxy compound used in the first mixing step was changed to the hydroxy compound shown in the following Table 6.

[Table 6]

| | | | Example 9 |
|---|---|---|---|
| Fine particles | Ultramicronized iron sesquioxide | | 0.1 |
| Hydroxy compound | Sucralose 134 μm | | 0.9 |
| High-molecular-weight dispersant | Povacoat | | 1 |
| Solvent | Water | | 19 |
| Evaluation of particle diameter | D50 | [nm] | 74 |
| | Span value | | 0.91 |

The terms in Table 6 have the following meanings. The numerical values in the tables denote the blending amounts (g).
Ultramicronized iron sesquioxide (trade name: Ultramicronized Iron Sesquioxide, manufactured by IoLiTec Ionic Liquids Technologies GmbH, average particle diameter D50: 20 to 40 nm).
Sucralose 134 μm: (trade name: J-600, manufactured by Tsuruya Chemical Industries, Ltd., average particle diameter D50: 134 μm).
Povacoat: (trade name: POVACOAT, manufactured by Daido Chemical Corporation, compound name: polyvinyl alcohol-acrylic acid-methyl methacrylate copolymer)
As shown in Tables 1 and 6, from a comparison between the liquid mixture of Example 5 and the liquid mixture of Example 9, it was found that the average particle diameter D50 and the span value of the ultramicronized iron sesquioxide were changed due to the average particle diameter D50 of sucralose. Specifically, with regard to sucralose, it was found that when sucralose having an average particle diameter D50 of 134 μm is used rather than sucralose having an average particle diameter D50 of 57 μm, the ultramicronized iron sesquioxide can be satisfactorily dispersed.

[Production 4 of liquid mixture]

(Example 10)

A liquid mixture of Example 10 was obtained by a method similar to the method for producing the liquid mixture of Example 2, except that with regard to the method for producing the liquid mixture of Example 2 as in [Production 1 of liquid mixture] described above, the ultramicronized iron sesquioxide was changed to yellow iron sesquioxide (trade name: Yellow Iron Sesquioxide, manufactured by Kishi Kasei Co., Ltd., average particle diameter D50: 100 to 500 nm).

(Comparative Example 7)

0.1 g of yellow iron sesquioxide (trade name: Yellow Iron Sesquioxide, manufactured by Kishi Kasei Co., Ltd., average particle diameter D50: 100 to 500 nm), 0.9 g of α-cyclodextrin (trade name: CELDEX A-100, manufactured by Nihon Shokuhin Kako Co., Ltd., average particle diameter D50: 36 μm), 1 g of the high-molecular-weight dispersant A, and 19 g of water were mixed using an ultrasonic homogenizer to obtain a liquid mixture of Comparative Example 7.

[Table 7]

| | | Example 10 | Comparative Example 7 |
|---|---|---|---|
| Fine particles | Yellow iron sesquioxide | 0.1 | 0.1 |
| Hydroxy compound | α-CyD | 0.9 | 0.9 |

(continued)

| | | | Example 10 | Comparative Example 7 |
|---|---|---|---|---|
| High-molecular-weight dispersant | Povacoat | | 1 | 1 |
| Solvent | Water | | 19 | 19 |
| Evaluation of particle diameter | D50 | [nm] | 152 | 277 |
| | Span value | | 0.87 | 3.52 |

The terms in Table 7 have the following meanings. The numerical values in the tables denote the blending amounts (g).

Yellow iron sesquioxide (trade name: Yellow Iron Sesquioxide, manufactured by Kishi Kasei Co., Ltd., average particle diameter D50: 300 nm).

α-CyD: α-Cyclodextrin (trade name: CELDEX A-100, manufactured by Nihon Shokuhin Kako Co., Ltd., average particle diameter D50: 36 μm).

Povacoat: (trade name: POVACOAT, manufactured by Daido Chemical Corporation, compound name: polyvinyl alcohol-acrylic acid-methyl methacrylate copolymer)

As shown in Table 7, it could be verified that in the liquid mixtures of Examples, the average particle diameter D50 and the span value of yellow iron sesquioxide were small as compared to the liquid mixtures of Comparative Examples, and the yellow iron sesquioxide was satisfactorily dispersed.

[Production 5 of liquid mixture]

(Example 11)

First mixing step:

0.1 g of titanium dioxide (trade name: Titanium Oxide FG, manufactured by Freund Corporation, average particle diameter D50: 550 nm) and 0.9 g of α-CyD: α-cyclodextrin (trade name: CELDEX A-100, manufactured by Nihon Shokuhin Kako Co., Ltd., average particle diameter D50: 36 μm) were subjected to a dry compositing treatment with a dry compositing apparatus (trade name: NOBILTA (registered trademark) Mini, manufactured by Hosokawa Micron Corporation) to obtain α-CyD coated with titanium dioxide (composite powder B).

Second mixing step:

1 g of the composite powder B, 1 g of the high-molecular-weight dispersant A (trade name: POVACOAT, manufactured by Daido Chemical Corporation, compound name: polyvinyl alcohol-acrylic acid-methyl methacrylate copolymer), and 19 g of water were mixed using an ultrasonic homogenizer (trade name: UD-200, manufactured by Tomy Seiko Co., Ltd.) to obtain a liquid mixture of Example 11, in which titanium dioxide coated with α-CyD was dispersed in water.

(Comparative Example 8)

0.1 g of titanium dioxide (trade name: Titanium Oxide FG, manufactured by Freund Corporation, average particle diameter D50: 550 nm), 0.9 g of α-CyD: α-cyclodextrin (trade name: CELDEX A-100, manufactured by Nihon Shokuhin Kako Co., Ltd., average particle diameter D50: 36 μm), 1 g of the high-molecular-weight dispersant A, and 19 g of water were mixed using an ultrasonic homogenizer to obtain a liquid mixture of Comparative Example 8.

[Measurement of particle diameter of fine particles]

The average particle diameters D10, D50, and D90 of titanium dioxide in the liquid mixture of each of the Examples were measured with a laser diffraction-type particle size distribution meter (trade name: LMS-2000, manufactured by Seishin Enterprise Co., Ltd.). The results are shown as "D50" in Table 8. In addition, the "span value" that can be determined by (D90 - D10) / D50 was also calculated, and the results are shown in Table 8.

[Table 8]

| | | | Example 11 | Comparative Example 8 |
|---|---|---|---|---|
| Fine particles | Titanium dioxide | | 0.1 | 0.1 |
| Hydroxy compound | α-CyD | | 0.9 | 0.9 |
| High-molecular-weight dispersant | Povacoat | | 1 | 1 |
| Solvent | Water | | 19 | 19 |
| Evaluation of particle diameter | D50 | [nm] | 62 | 313 |
| | Span value | | 1.08 | 1.28 |

As shown in Table 8, it could be verified that in the liquid mixtures of Examples, the average particle diameter D50 and the span value of titanium dioxide were small as compared to the liquid mixtures of Comparative Examples, and the titanium dioxide was satisfactorily dispersed.

Since the liquid mixture of Comparative Example 8 was obtained by simply mixing each of the raw materials using an ultrasonic homogenizer, the aggregation of the titanium dioxide could not be crushed, and the average particle diameter D50 of the titanium dioxide had a large value.

[Production 6 of liquid mixture]

(Examples 12 to 16)

Liquid mixtures of Examples 12 to 16 were obtained in the same manner as in the case of the liquid mixture of Example 2, except that the contents of ultramicronized iron sesquioxide (trade name: ultramicronized iron sesquioxide, manufactured by IoLiTec Ionic Liquids Technologies GmbH, average particle diameter D50: 20 to 40 nm), α-CyD: α-cyclodextrin (trade name: CELDEX A-100, manufactured by Nihon Shokuhin Kako Co., Ltd., average particle diameter D50: 36 μm), Povacoat: (trade name: POVACOAT, manufactured by Daido Chemical Corporation, compound name: polyvinyl alcohol-acrylic acid-methyl methacrylate copolymer), and water were changed as shown in Table 9.

[Measurement of particle diameter of fine particles]

The average particle diameters D10, D50, and D90 of the ultramicronized iron sesquioxide in the liquid mixture of each Example were measured with a laser diffraction-type particle size distribution meter (trade name: LMS-2000, manufactured by Seishin Enterprise Co., Ltd.). The results are shown as "D50" in Table 9. In addition, the "span value" that can be determined by (D90 - D10) / D50 was also calculated, and the results are shown in Table 9.

From the viewpoint of facilitating comparison, the liquid mixture of Example 2 shown in the above-described Table 1 is shown again in Table 9.

[Table 9]

| | | Example 12 | Example 2 | Example 13 | Example 14 | Example 15 | Example 16 |
|---|---|---|---|---|---|---|---|
| Fine particles | Ultramicronized iron sesquioxide | 0.1 | 0.1 | 0.1 | 0.1 | 0.1 | 0.1 |
| Hydroxy compound | α-CyD | 1.8 | 0.9 | 0.56 | 0.4 | 0.9 | 0.9 |
| High-molecular-weight dispersant | Povacoat | 1 | 1 | 1 | 1 | 0.4 | 0.2 |
| Solvent | Water | 19 | 19 | 19 | 19 | 19.6 | 19.8 |

(continued)

| | | | Example 12 | Example 2 | Example 13 | Example 14 | Example 15 | Example 16 |
|---|---|---|---|---|---|---|---|---|
| Evaluation of particle diameter | D50 | [nm] | 82 | 76 | 100 | 385 | 82 | 81 |
| | Span value | | 0.898 | 0.889 | 8.585 | 5.376 | 0.864 | 6.546 |

As shown in Table 9, it could be verified that even in a case where the content of each component was changed, the liquid mixtures of Examples had a small average particle diameter D50 and a small span value of the ultramicronized iron sesquioxide, and the ultramicronized iron sesquioxide was satisfactorily dispersed.

Furthermore, it could be verified that among them, particularly, the liquid mixtures of Examples 2, 12, and 15 had a small average particle diameter D50 and a small span value of the ultramicronized iron sesquioxide, and the ultramicronized iron sesquioxide was more satisfactorily dispersed.

Thus, preferred Examples of the present invention have been described above; however, the present invention is not intended to be limited to these Examples. Additions, omissions, substitutions, and other modifications can be made without departing from the gist of the present invention. The present invention is limited not by the above description but only by the scope of the appended claims.

## Claims

**1.** A method for producing a liquid mixture, the method comprising:

a first mixing step of mixing fine particles and a hydroxy compound to obtain a hydroxy compound coated with fine particles; and
a second mixing step of mixing the hydroxy compound coated with fine particles with a solvent to obtain a liquid mixture in which fine particles coated with a hydroxy compound are dispersed in the solvent.

**2.** The method for producing a liquid mixture according to Claim 1,
wherein the hydroxy compound is one or more compounds selected from the group consisting of a sugar, a vitamin P derivative, and a salt of a vitamin P derivative.

**3.** The method for producing a liquid mixture according to Claim 1 or 2,
wherein a high-molecular-weight dispersant is further mixed in the second mixing step.

**4.** The method for producing a liquid mixture according to any one of Claims 1 to 3,
wherein the average particle diameter D50 of the fine particles in the first mixing step is 1 nm or greater and 900 nm or less, and the average particle diameter D50 of the hydroxy compound is 30 $\mu$m or greater and 300 $\mu$m or less.

**5.** The method for producing a liquid mixture according to any one of Claims 1 to 4,
wherein the average particle diameter D50 of the fine particles in the second mixing step is 410 nm or less.

**6.** The method for producing a liquid mixture according to any one of Claims 1 to 5,
wherein in the first mixing step, the use amount of the hydroxy compound is 200 parts by mass or greater and 2,500 parts by mass or less with respect to 100 parts by mass of the fine particles.

**7.** A liquid mixture in which fine particles coated with a hydroxy compound are dispersed in a solvent,
wherein the hydroxy compound is one or more compounds selected from the group consisting of D-mannitol, $\alpha$-cyclodextrin, HP-$\beta$-cyclodextrin, methyl hesperidin, and sucralose.

**8.** A solid composition coated with the liquid mixture according to Claim 7.

**9.** A solid pharmaceutical composition coated with the liquid mixture according to Claim 7.

**10.** A solid composition created by using the liquid mixture according to Claim 7.

**11.** A liquid mixture comprising:

a hydroxy compound; and
fine particles,
wherein the average particle diameter D50 of the fine particles is 200 nm or less, and the span value of the fine particles is 9 or less.

FIG. 1

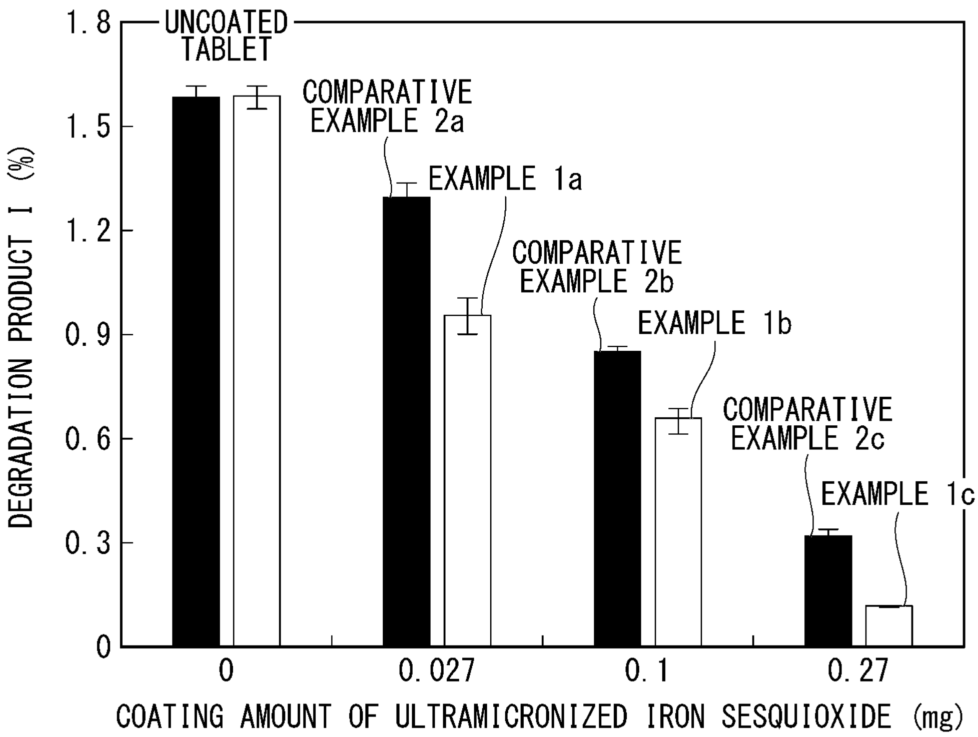
UNCOATED TABLET
COMPARATIVE EXAMPLE 2a
EXAMPLE 1a
COMPARATIVE EXAMPLE 2b
EXAMPLE 1b
COMPARATIVE EXAMPLE 2c
EXAMPLE 1c
DEGRADATION PRODUCT I (%)
1.8
1.5
1.2
0.9
0.6
0.3
0
0.027
0.1
0.27
COATING AMOUNT OF ULTRAMICRONIZED IRON SESQUIOXIDE (mg)

FIG. 2

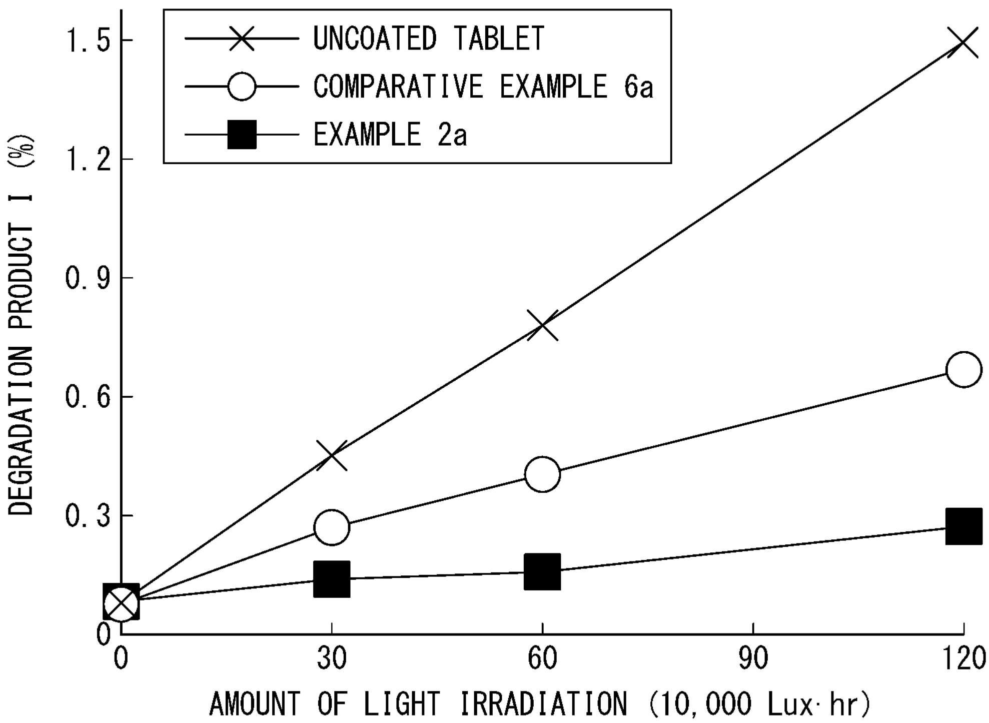
UNCOATED TABLET
COMPARATIVE EXAMPLE 6a
EXAMPLE 2a
DEGRADATION PRODUCT I (%)
1.5
1.2
0.9
0.6
0.3
0
30
60
90
120
AMOUNT OF LIGHT IRRADIATION (10,000 Lux·hr)

**INTERNATIONAL SEARCH REPORT**

International application No. **PCT/JP2022/037812**

**A. CLASSIFICATION OF SUBJECT MATTER**

***A61K 47/26***(2006.01)i; ***A61K 9/14***(2006.01)i; ***A61K 9/32***(2006.01)i; ***A61K 9/36***(2006.01)i; ***A61K 31/4422***(2006.01)i; ***A61K 47/02***(2006.01)i; ***A61K 47/40***(2006.01)i; ***A61K 47/58***(2017.01)i
FI: A61K47/26; A61K9/14; A61K47/40; A61K9/36; A61K9/32; A61K47/58; A61K47/02; A61K31/4422

According to International Patent Classification (IPC) or to both national classification and IPC

**B. FIELDS SEARCHED**

Minimum documentation searched (classification system followed by classification symbols)

A61K47/26; A61K9/14; A61K9/32; A61K9/36; A61K31/4422; A61K47/02; A61K47/40; A61K47/58

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2017-509691 A (BPSI HOLDINGS, LLC) 06 April 2017 (2017-04-06) entire text, in particular, paragraphs [0008], [0009], [0012], [0013], [0021], [0022], [0025], [0026], [0028], [0035]-[0038], [0045]-[0053] | 1-11 |
| Y | | 1-11 |
| X | WO 2020/080380 A1 (TOWA PHARMACEUTICAL CO LTD) 23 April 2020 (2020-04-23) entire text, in particular, paragraphs [0001], [0008], [0014], [0016], [0018], [0019], [0023]-[0031], [0035]-[0061] | 1-7, 11 |
| Y | | 1-11 |
| X | JP 2011-057856 A (JGC CATALYSTS & CHEMICALS LTD) 24 March 2011 (2011-03-24) paragraphs [0001], [0002], [0007], [0013], [0014], [0016]-[0018], [0022], [0026]-[0030], [0056]-[0060], [0075]-[0080] | 7, 8, 10, 11 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 December 2022** | **10 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/037812**

| Patent document cited in search report | Publication date (day/month/year) | Patent family member(s) | Publication date (day/month/year) |
|---|---|---|---|
| JP 2017-509691 A | 06 April 2017 | US 2017/0035697 A1<br>paragraphs [0007], [0008], [0017], [0018], [0026], [0027], [0030], [0032], [0038]-[0041], [0047]-[0051]<br>EP 3096891 A1<br>WO 2015/112400 A1 | |
| WO 2020/080380 A1 | 23 April 2020 | US 2021/0346301 A1<br>paragraphs [0002], [0008], [0014], [0017], [0019]-[0021], [0023]-[0037], [0039]-[0060]<br>EP 3868366 A1<br>WO 2020/080380 A1 | |
| JP 2011-057856 A | 24 March 2011 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021166788 A
- JP 6186032 B


### Non-patent literature cited in the description


- Japanese Pharmacopoeia, the Japanese Pharmaceutical Codex, Japanese Pharmaceutical Excipients. Yakuji Nippo, Ltd, 2018
- Japanese Pharmaceutical Excipients Directory. Yakuji Nippo, Ltd, 2021
- Handbook of Pharmaceutical Excipients. Pharmaceutical Press, 2012
- Japanese Standards of Cosmetic Ingredients. Yakuji Nippo, Ltd, 1984
- Japanese Cosmetic Ingredients Codex. Yakuji Nippo, Ltd, 1993
- Supplement to the Japanese Cosmetic Ingredients Codex. Yakuji Nippo, Ltd, 1993
- Comprehensive Licensing Standards of Cosmetics by Category. Yakuji Nippo, Ltd, 1993
- Dictionary of Cosmetic Ingredients. Nikko Chemicals Co., Ltd, 1991
- International Cosmetic Ingredient Dictionary and Handbook. CTFA, 2002, vol. 1-4